# EUROPEAN PATENT SPECIFICATION

(11) **EP 2 443 577 B1**
(45) Date of publication and mention of the grant of the patent: **09.09.2020**
(21) Application number: 10741895.6
(22) Date of filing: 21.06.2010
(51) Int. Cl.: G16H 20/10, G16H 20/60, G16H 40/63

(54) **METHODS AND SYSTEMS FOR ADVISING PEOPLE WITH DIABETES**
VERFAHREN UND SYSTEME ZUM BERATEN VON PERSONEN MIT DIABETES
PROCÉDÉS ET SYSTÈMES DESTINÉS À CONSEILLER DES PERSONNES DIABÉTIQUES

(30) Priority: 19.06.2009 US 487754
(43) Date of publication of application: 25.04.2012
(73) Proprietor: F. Hoffmann-La Roche AG, 4070 Basel (CH); Roche Diabetes Care GmbH, 68305 Mannheim (DE)
(72) Inventor: GALLEY, Paul, Cumberland IN 46229 (US); ZIVITZ, Maury, Indianapolis IN 46250 (US); PRICE, John, McCordsville IN 46055 (US); GREENBURG, Alan, Indianapolis IN 46256-1181 (US); LONG, James, Fishers IN 46038 (US)
(74) Representative: Bittner, Thomas L.
(86) International application number: PCT/EP2010/003720
(87) International publication number: WO 2010/145840

(56) References cited:
- WO-A2-2007/028271
- US-A1- 2005 197 553
- US-B1- 6 188 648

## Description

### TECHNICAL FIELD

The present invention generally relates to methods and systems for advising persons with diabetes and, specifically, for advising based on their recommended therapy.

### BACKGROUND

As background, people suffer from either Type I or Type II diabetes in which the sugar level in the blood is not properly regulated by the body. As a consequence, doctors or other health care professionals often recommend a therapy for the person with diabetes to follow. This therapy may be based on the type of diabetes, the severity of the diabetes, and the personal characteristics of the person with diabetes.

The recommended therapy may comprise a number of individual tasks, each of which should be performed at specific times or at specific events. For example, one task may advise the person with diabetes to take basal insulin or oral medication twice per day. The timing and the details of the task may depend on real-time data, such the time and level of the last blood glucose (bG) level result, the time and amount of the previous meal, and so forth. Some of the various therapeutic tasks that should be performed can be complicated. Other tasks may only be performed occasionally and may be difficult for the person with diabetes to remember.

The document WO 2007/028271 A2 discloses a system, tool, device method and program allowing to characterize the relevance of errors of parameters affecting glucose concentration on a postprandial glucose concentration outcome for a person with diabetes mellitus. It describes in detail the effects of potential errors of parameters affecting glucose concentration on postprandial glucose concentration values within the clinically relevant glucose range. Treatment advice may be given based on the prediction of postprandial blood glucose.

US 2005/0197553 A1 discloses a portable diabetes management device that calculates medication dosages from glucometer readings and data such as food intake entered by the patient, according to a management plan that can be personalized by a health-care professional using a template. The device may also store and communicate past data and plan revisions.

### SUMMARY

It is against the above background that embodiments of the present invention simplify the ability of a person with diabetes to follow a recommended therapy by providing methods and/or systems which advise the person with diabetes to perform a task based on the recommended therapy.

The invention is defined in the independent claims, according to which a method and a portable electronic device for advising a person with diabetes are provided. Preferred embodiments are defined in the dependent claims.

### BRIEF DESCRIPTION OF THE DRAWINGS

The embodiments set forth in the drawings are illustrative and exemplary in nature and not intended to limit the inventions defined by the claims. The following detailed description of the illustrative embodiments can be understood when read in conjunction with the following drawings, where like structure is indicated with like reference numerals and in which:
FIG. 1 depicts a method of providing clinical advice for diabetes according to one or more embodiments shown and described herein;
FIG. 2 depicts a portable, electronic device according to one or more embodiments shown and described herein;
FIG. 3 depicts a block diagram of an advising system according to one or more embodiments shown and described herein; and
FIG. 4 depicts a personal computer according to one or more embodiments shown and described herein.

### DETAILED DESCRIPTION

The invention is defined by the appended claims. Other embodiments herein described have to interpreted as examples.

The embodiments described herein generally related to methods and systems for advising persons with diabetes. The systems discussed include portable electronic devices, such as cellular phones, smart phones, and the like. Other systems may include personal computers, for example desktop or laptop computers. The advice provided by the embodiments of the present invention is not intended to replace the advice given by a physician or other health care professional. Instead, the advice is merely intended to assist the person with diabetes in adhering to his specific therapy for treating diabetes which was recommended to him by his health care professional.

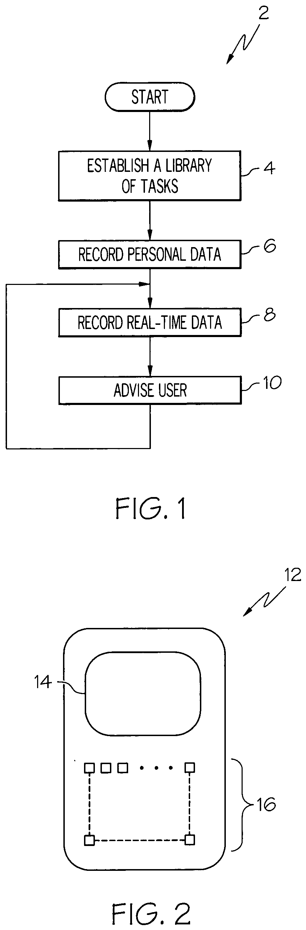

An exemplary method for advising persons with diabetes is depicted in FIG. 1. The method 2 comprises the steps of establishing a library of tasks 4, recording personal data, 6, recording real-time data 8, and advising the user to perform a task 10. In step 4, a library of tasks is established. This library may also contain settings as well. There exists a large number of tasks which may be performed by a person with diabetes as part of his individual therapy. For example, people suffering from Type I diabetes may have a different recommended therapy than people suffering from Type II diabetes. Likewise, personal characteristics, such as age and weight, may also affect the therapy. Thus, from a large group of potential tasks, each individual person with diabetes may employ a smaller subset of these tasks for his specific therapy. Furthermore, a particular task may be customized for each person. As an example, the recommended amount of insulin to be taken may be unique for a particular person with diabetes. Accordingly, the recommended therapy for each person may be unique, and thus the library of tasks (and settings), which is related to the person's therapy, is a subset of a larger group of general tasks.

In one embodiment, the method 2 may be performed on a portable electronic device, such as a cellular phone, a personal digital assistant, or a smart phone. In this case, the tasks selected to be included in the library may correspond to the characteristics of the portable electronic device. That is, tasks may be omitted from the library if they are not appropriate for the portable electronic device. For example, a task which displays a large amount of data may not be appropriate for the portable electronic device since the display on such a device is typically small, and it may be difficult for the user to read or interpret the data on such a small display. In another example, tasks which require a large amount of data to be inputted may not be appropriate for the portable electronic device since it may be cumbersome to enter the data using the inputting means (e.g., keyboard) of the device. Thus, when establishing the library of tasks, the method may select tasks based on the recommended therapy as well as the characteristics of the portable electronic device.

In step 6, personal data about the person with diabetes may be recorded. This data may represent physical characteristics of the person, such as age, weight, height, and so forth. Other characteristics may be recorded as well, including target weight, target bG level, etc. Although a health care professional may have used many of these characteristics while determining the person's therapy, they may also be used to provide advice to the person, particularly since some of these characteristics may change slightly over time. For example, a person's weight may be a factor in determining his overall therapy; however, weight may also be used to refine the advice given by the method 2, since fluctuations in weight may alter the content of the advice. Thus, one embodiment of the method may record personal data about the person in order to provide improved advice. A specific set of personal data is related to establishing the appropriate individual settings to customize the library of tasks to the specific goals and therapy plans agreed between the person with diabetes and his healthcare professional.

In step 8, the method 2 may record real-time data about the person with diabetes. Real-time data is information which can change relatively quickly, such as over the course of a few minutes or a few hours. Data recorded at this step may include the bG level, the amount of insulin taken, or whether a meal was eaten. Data may be manually input or may come from one or more devices or data repositories. Because of the nature of the data, a time stamp may be required for each piece of data. For example, the bG level result and the time that the bG level was taken may be recorded by the method. In some cases, these multiple pieces of information concerning a specific event may be recorded at different times. Although it is preferable that all information be recorded at the same time and as temporally proximate to the event as possible, the recording of information at a later time may be convenient for the user. As an example, it may be convenient for the user to record the fact that he ate a meal without entering the details (e.g., what exact time, how many carbohydrates, how much sugar, etc.) In this case, the method 2 may temporarily substitute unconfirmed default values for the unknown data which are typical for that person (e.g., based on past meals). Different downstream processing may occur driven by the quality of the real-time data and timestamps.

Continuing to refer to FIG. 1, in step 10, the method 2 may advise the user to perform a task, based on the tasks in the library, the personal data, and the real-time data. The types of advice which may be given are describe herein below. The advice given should be consistent with the recommended therapy for that person. The method 2 does not require the user to follow the advice, but merely offers a convenient and easy way for the user to follow his recommended therapy. When advice is given to the user at step 10, the method 2 may return to step 8 and await additional real-time data before providing further advice.

The method 2 may advise a user based, at least in part, on a real-time clock. A real-time clock is a method of tracking the time and day; it is, in essence, a clock and a calendar. Some of the tasks may not require a real-time clock to provide advice. However, other tasks may require a real-time clock. For example, a task which reminds the user to inject basal insulin may require a real-time clock since this is typically done at a specific time of the day. Other tasks may require the expiration of a certain number of days before advising the user again to take some action. Thus, the advice given at step 10 may be based on the personal data, the real-time data, and a real-time clock.

FIG. 2 depicts an exemplary device on which the method 2 may be performed. The device shown may be a portable electronic device 12, such as a cellular phone, a portable digital assistant, or a smart phone. The device 12 may have a display 14 and a means for inputting data 16, such as a keyboard (as shown) or touch screen. Other means for inputting data are contemplated, including wired and wireless means. As an example, the device 12 may receive bG level results from a bG meter through a wireless Bluetooth connection.

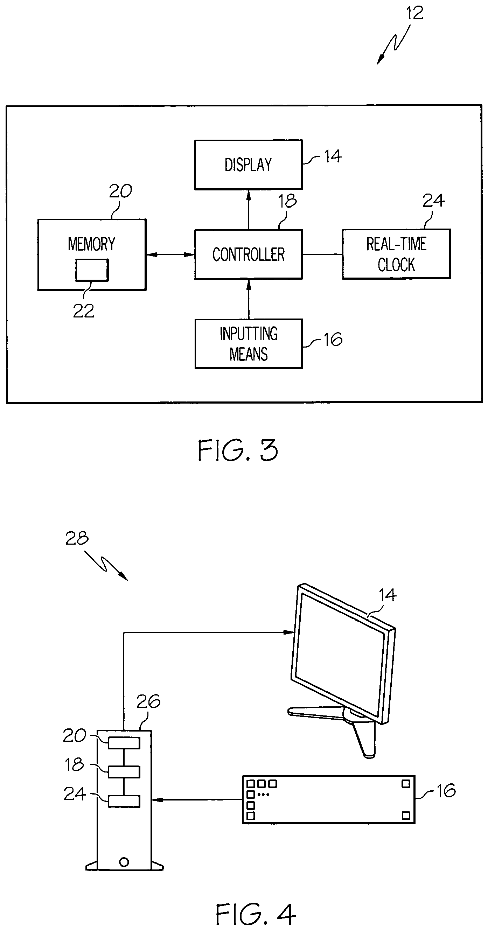

FIG. 3 illustrates a block diagram of a portable electronic device 12. In addition to the display 14 and the inputting means 16, the device 12 may contain a controller 18 and a memory 20. The memory 20 may contain a library of tasks 22, which may be established as previously described herein. The controller 18 may be in communication with the other components and may comprise a microprocessor or similar element. The method of advising a person with diabetes, i.e., method 2, may be implemented by a computer program which is executed by the controller 18. The computer program may be written in assembly language, the "C" language, the "C++" language, or other suitable programming language. The computer program may reside in the memory 20 or the controller 18. The real-time clock 24, if used, may be coupled to the controller 18.

Another embodiment of the system is shown in FIG. 4. A personal computer 28 may also advise persons with diabetes according to method 2. The personal computer may be either a desktop computer (as shown in FIG. 4) or a laptop computer or web-based application. The personal computer may comprise a display 14, a means for inputting data 16, and a processing unit 26. The processing unit 26 may comprise a controller 18, memory 20, and a real-time clock 24. As previously discussed herein, the memory 20 may contain the library of tasks (and settings as well). In addition to personal computers, it is contemplated that other types of systems may be used for advising persons with diabetes, including yet-to-be-discovered systems.

The types of advice for diabetes may fall into five basic categories. First, there may be simple reminders. This may help empower the patient through visual, audible and/or vibratory reminders that suggest the user test, enter, or view data. These are typically simple, user settable reminders that are defaulted to appropriate clock times, time blocks, or intervals after specific entry-based conditions. The second category contains warnings and teachable moments, which may provide the user with practical and actionable information on demand. Information within this category may be contextually tied to measured bG results, documentation of health and exercise conditions, or in a frequently-asked questions (FAQ) type of venue. The formats may include real-time contextual warnings and alerts, on-demand information screens, or video content. The third category consists of assessments and reports. This may provide the user with real-time or on-demand assessment of the bG result within a notebook entry against its appropriate target range as well as provided retrospective data graphical reporting, especially relating to cause and effect relationships. The fourth category includes focused testing which may help empower the person with diabetes through visual, audible and/or vibratory reminders that suggest the user test and enter data to fulfill a specific sequence or chain of events. The fifth category comprises recommended doses and supplements. Other categories may be used as well. These five categories will be discussed in detail below.

The first category of tasks is simple reminders, which may advise the person with diabetes to perform a test, enter data, or view data. These are typically based on clock times, time blocks, or intervals after specific entry-based conditions. Exemplary tasks will now be described. A "Basal" reminder task may be a recurring, once-a-day (or possibly twice-a-day) reminder to take basal insulin or oral medication. A "Fasting Plasma Glucose" reminder may be a once-a-day reminder to perform a glucose test. A "Meal Entry" reminder may advise the person with diabetes to eat a meal and/or take medication with the meal. A "Post-prandial" reminder may be a conditional occurrence reminder which is triggered when a user declares (i.e., records) a meal and reminds the user to perform a bG test after an appropriate interval (e.g., 90 minutes) according to his recommended therapy. A "High-bG Recovery" reminder may be a conditional occurrence reminder triggered when a user enters a bG result that is above a defined threshold and reminds the user to perform a bG test after the appropriate interval. This reminder may be used to confirm whether a therapeutic option was inadequate or may be part of a sick day management plan.

Continuing with examples of simple reminders, a "Hypo Recovery" reminder may be a conditional occurrence reminder triggered when a user enters a bG result that is below a defined threshold and reminds the user to perform a bG test after the appropriate interval. This may be used to confirm whether a therapeutic option was inadequate (over treatment or under treatment) or may be part of a hypoglycemia treatment plan. An "Exercise bG" reminder may be a conditional occurrence reminder triggered when a user records an exercise record that reminds the user to perform a bG test after the appropriate interval. This reminder may also be implemented as an informational screen on the display which suggests that the user test his bG level after exercise or at regular intervals during extended exercise. Such reminders may also assist the user in appropriately documenting bG results that occur before, during, or after exercise. A "Meal Intake" reminder may be a conditional reminder about when to consume a meal in order to allow the medication to take effect. As the interval between medication and the meal in increased, or as the complexity of rules makes such a time variable, a reminder to eat serves a practical purpose to help maintain optimal therapy as well as to minimize user concerns about hypoglycemia because of forgotten meals. This reminder may also allow for an easier documentation of meal consumption times and the possibility to review the diary subsequently for appropriate medication-meal intervals.

Continuing further with examples of simple reminders, a "Medication" reminder may be a recurring, conditional reminder triggered after a user enters a new medication and reminds the user to take the medication. This reminder may be enabled for some time period such as 2 weeks and may behave similarly to the "Meal Entry" reminder. This may assist the user in successfully attaining a habit of adhering to a new medication. A "Travel" reminder may be a conditional occurrence reminder triggered when a user indicates a date and time for future travel and provides the user travel tips. A "Weekly Data Assessment" reminder may be a recurring, once-a-week (occasionally once-every-three-days) reminder to review graphs or reports and may help guide users in assessing their data at an appropriate and regular interval, depending on the therapy. Finally, a "Doctor's Office Visit" reminder may be a single occurrence reminder, based on the date and time, which is triggered after a user schedules a visit to a health care professional or other diabetes team member and reminds the user of the visit. This may also be a trigger for enabling informational messages to be display indicating that the user should prepare for the visit, such as printing out data or performing focused testing or other higher density data collection. This reminder may also act as a time marker to help visualize and compare the change in glycemic control over the relevant time periods. Any of these simple reminders may be triggered at a clock time or an offset within a time block, depending on whether it is a real-time reminder or a reminder that the entry was missed or forgotten to be entered.

The second category of tasks is warnings and teachable moments, which may advise the person with diabetes with practical and actionable information on demand. The information provided may be contextually tied to measured bG results, documentation of health and exercise conditions, or in a frequently-asked questions (FAQ) type of venue. The formats may include real-time contextual warnings and alerts, on-demand information, or video content. The video content may include information relevant to current product offerings (e.g., topics from Getting Started Guides, Troubleshooting Guides, or training videos) of a particular vendor. Exemplary tasks in this category are described below.

A "High bG" warning may advise the user to monitor bG levels more frequently during illness and to perform a ketone test each time a measured bG level is greater than a certain value, such as 250 mg/dL. A "Sick Day" warning may also advise the user to monitor bG levels more frequently. In addition, if the user is hyperglycemic, the warning may advise the user when to contact a health care provider, advise the user of the bG goals and the use of supplemental short-acting insulin, advise the means to suppress fever and treat infection, or advise the initiation of an easily-digestible liquid diet containing carbohydrates and salt. The user is usually advised not to discontinue insulin and to seek professional advice early in the course of the illness. A "Hypo bG" warning may advise the user to monitor bG levels more frequently due to hypoglycemia. This monitoring may include both pre-prandial and 2-hour post-prandial bG levels as well as occasionally 2:00 am to 3:00 am bG levels. The warning may also indicates the symptoms of hypoglycemia, such as shakiness, dizziness, sweating, headache, pale skin color, and so forth. The warning may also suggest possible treatments, such as ingestion of 15-20 grams (g) of glucose or ingestion of food containing carbohydrates.

Continuing with examples of warnings and teachable moments, a "Monitor Side Effects" teachable moment may make the user aware of potential side effects when beginning a new medication. A "Travel Tips" teachable moment may provide the user with possible ways to improve the travel experience. A "Clock Time Change" warning may indicate to the user that the clock has changed, either due to the periodic adjustments of clocks or due to travel across time zone boundaries. A "Hospital Stay Preparation" task is a teachable moment which could help the patient learn about necessities for an in-patient or out-patient procedure. Finally, a "Recurring Hypoglycemia" warning may advise the user that he is more prone to a recurrence of hypoglycemia within 24 hours of having hypoglycemia. These are only exemplary warnings and teachable moments, and others are also contemplated.

The third category of tasks is assessments and reports, which may provide the person with diabetes real-time or on-demand assessments of the bG result within a logbook entry against its appropriate target range based on his recommended therapy. Furthermore, this may provide the user with improved retrospective data graphical reporting, especially relating to cause-and-effect relationships. The person with diabetes may be able to examine the data visually and make appropriate therapeutic decisions which are in line with the goals and targets provided to him by his health care professional. Examples of assessments and reports are described below.

An "Autolabel" assessment may, after declaring a meal and during the entry of a data record, review prior history to determine whether the entry can be considered post-prandial. Such an automatic assessment, suggesting a contextually appropriate label for data, can be more useful than the industry's traditional time-block-based approaches. An "Assess bG (fasting)" task may permit the user, in the context of entering and reviewing data records, to be able to see how the specific entered bG result compared to a recommended fasting bG target range. The fasting target range may default to the pre-meal target range, unless the user specifies otherwise. There are guidelines from the major organizations for fasting bG targets. This task may provide immediate feedback on entered or later reviewed data compared to expectations and may encourage people to be more engaged with their therapy. An "Assess bG (pre-meal)" task may permit the user, in the context of entering and reviewing data records, to be able to see how the specific entered bG result compared to a defined pre-prandial bG target range. An "Assess bG (post-meal)" task may allow the user, in the context of entering and reviewing data records, to be able to see how the specific entered bG result compared to a defined post-prandial bG target range. There are guidelines from the major organizations for post-prandial bG targets. An "Assess bG (night)" task may, in the context of entering and reviewing data records, permit the user to be able to see how the specific entered bG result compared to a defined bedtime or 3:00 am bG target range. The bedtime target range could default to the pre-meal target range plus an elevation to be aligned with organizational guidelines, unless the user specifies otherwise.

Continuing with additional tasks in the assessments and reports category, an "Event-based Report" may help the user better visualize and understand potential cause and effect relationships within their data and permit the user to review the time relationship of glucose values (and possibly other variables) in the vicinity (e.g., +/- 6 hour window) of a selected type of event. A "Goals Report" may allow the user to review the longer-term assessment of goals related to glucose, meals, exercise and medication. A similar goals view may be also available for other non-glucose health records (including HbA1c, blood pressure, lipids profile, and body weight or BMI). Other tasks may assess the adherence of the user to new medication, report on specific bG patterns, report on the financial cost of using bG test strips, and assess the complexity of the user's medication regimen. Other types of assessments and reports are contemplated.

The fourth category of tasks is related to structured or focused testing. These types of tasks may help remind the user, through visual, audible and/or vibratory means, to test and/or record data to fulfill a specific sequence of events. The method may recommend (or even initiate) focused testing; however, it may also be initiated by the user. Examples of such tasks will now be described. An "Overnight" focused test may be used to confirm the overnight basal dosage. A series of reminders may be triggered to take a bG measurement at bedtime, at mid-sleep, and in the morning. In one embodiment, the focused test would only begin if the bedtime bG result were within target. It would end if any insulin, carbohydrates or alternate state were activated, or the bG result changed by more than 30 mg/dL from the starting point. A "Skipped Meal" focused test may be designed to confirm the daytime basal dosage. A series of reminders may be triggered at pre-meal to take bG measurements at a regular interval until the next meal. In one embodiment, this focused test may only begin if the pre-meal bG level were within target. It would end if any insulin, carbohydrates or alternative state were activated, or the bG level changed by more than 30 mg/dL from the starting point. A "Fasting" focused test may be designed to confirm the daytime basal dosage. A series of reminders may be triggered at the first reading of the day to take bG measurements at a regular interval until the next meal. In one embodiment, the focused test would begin if the fasting bG level were within target. It would end if any insulin, carbohydrates or alternate state were activated, or the bG changed by more than 30 mg/dL from the starting point. Any of these focused tests may enhance the adherence of collecting information useful in assessing and adjusting the basal dosage.

Continuing with examples of tasks in the fourth category, a "Meal" focused test may be used to confirm the match of the meals with the medication. A series of reminders may be triggered at pre-meal to take bG measurements at a regular interval until the next meal. This may help the person with diabetes assess and adjust the prandial therapy. In one embodiment, the experiment would only begin if the pre-meal bG level were within target. It would end if any insulin, carbohydrates, or alternate state were activated. This task may additionally verify that the basal insulin is properly established before testing meals. A "High bG" focused test may be used to confirm the bG correction therapy. A series of reminders could be triggered (e.g., when a user records a bG level result that is above a defined threshold) to take bG measurements at a regular interval until the next meal or bedtime. This may enhance the adherence of collecting bG data useful in assessing and adjusting the bG correction therapy. It may also be performed instead of the "High bG Recovery" reminder. In one embodiment, this task would end if any insulin, carbohydrates, or alternate state were activated, or the bG dropped below the target (hypoglycemia). The "Hypo Surveillance" focused test may help confirm that the user recovered from the hypoglycemia therapy. A series of reminders may be triggered (e.g., when a user records a bG level result that is below a defined threshold) to take bG measurements at a regular interval until the next meal or bedtime. This may enhance the adherence of collecting bG data useful in assessing and adjusting the bG correction therapy. It may also be performed instead of the "Hypo Recovery" reminder. In one embodiment, this task would end if any insulin, carbohydrates, or alternate state were activated, or the bG level remained hypoglycemic.

Continuing further with examples of tasks in the fourth category, a "Pre- and Post-bG Day Profile" focused test may be designed to confirm the adequacy of the overall therapy. The task may provide a shortcut to remind the user after each daytime meal to take a post-prandial bG measurement. This may allow the user to collect sufficient bG data to create a "six or seven point profile." In one embodiment, the focused test could include an optional meal/bedtime or mid-sleep time block entry reminder. This task may be performed instead of any "Post-prandial" reminder. Chaining together multiple pre- and post-bG day profiles may enable the 3-Day Snapshot or the temporary testing mode. A "Temporary Testing Mode" focused test may be used to provide comprehensive overview of the therapy prior to a visit to a health care professional or following therapy change. This task may include a shortcut to remind the user after each daytime meal to take a post-prandial bG measurement. A multi-day implementation of the pre- and post-prandial bG measurement may be used. This may help the user collect bG information useful in assessing and adjusting the glycemic therapy. It may include an optional weekly mid-sleep reminder and may be performed instead of any "Post-prandial" reminder. In one embodiment, once a test frequency criterion is met, the task may stop requesting the bG results for specific pre- or post-meal or fasting conditions. A "Temporary Adjusting Mode" focused test may be designed to provide temporary fine-tuning adjustments following a change in therapy (e.g., the addition of a single, fixed prandial insulin at supper for users with Type II diabetes). A shortcut may be used to adjust therapy after user indication of new medication (e.g., Symlin).

Continuing further with examples of tasks in the fourth category, a "3-Day Snapshot" focused test may be an extension of the "Pre- and Post-bG Day Profile" task and may cover a three-day period. This task may confirm the adequacy of the overall therapy for hypoglycemia, fasting, and pre- and post-prandial hyperglycemia. Finally, a "CM Alternative" focused test may be design to confirm the adequacy of the overall therapy. This task may be a multi-day implementation of the "Meal" focused test. Other types of tasks in this category are contemplated as well.

The fifth category of tasks is recommended doses and supplements. These types of tasks may help the user determine the adequacy of the dosage of medication he is taking as well as the timing of taking the medication. This category may concern primarily insulin, although other types of medication are contemplated as well. Tasks in this category may help the user determine whether the dosage and/or timing is adequate for fixed basal insulin, a basal supplement, a fixed non-insulin basal medication, fixed prandial insulin, computed prandial, computed bG correction, computed bolus type, fixed fast-acting carbohydrates, computed fast-acting carbohydrates, computed supplements, fixed carbohydrate intake delay (CID), computed carbohydrate intake delay, fixed new medications, and computed new medications. Other types of medications may be monitored by the tasks as well.

It should now be understood that the modules described herein may be embedded in a host so as to provide the host with the capability of measuring blood glucose levels or interstitial glucose levels. In addition the host may record and control of therapeutic delivery of medication. The module may be operable to be embedded in the host and to communicate to the host through the host interface. The module is operable to perform a blood glucose measurement and to communicate the measurement result to the host via the host interface.

While particular embodiments and aspects of the present invention have been illustrated and described herein, various other changes and modifications may be made. Moreover, although various inventive aspects have been described herein, such aspects need not be utilized in combination. It is therefore intended that the appended claims cover all such changes and modifications.

## Claims

1. A method for advising a person with diabetes, the method performed on a portable electronic device and the method comprising:
- establishing, in a memory of the portable electronic device, a library of one or more tasks, wherein each task in the library is related to a therapy recommended to the person with diabetes and the one or more tasks are a subset from a large group of potential tasks, the subset being specific to the therapy recommended to the person;
- recording personal data received via an inputting means of the portable electronic device, the personal data representing characteristics of the person related to the person's diabetic condition;
- recording real-time data received via the inputting means of the portable electronic device, the real-time data being related to the person's diabetic condition; and
- advising the person, via a display of the portable electronic device, to perform a task from the library, based on the personal data and the real-time data,
**characterized in that** the tasks correspond to the characteristics of the portable electronic device **in that** tasks are omitted from the library if they are not appropriate for the portable electronic device.

2. The method of claim 1, wherein the task being advised is injecting basal insulin, taking a blood glucose measurement, recording the person's meal data and time, taking medication, or visiting the doctor.

3. The method of claim 1 or 2, wherein the portable electronic device is a cellular phone, a personal digital assistant, or a smart phone.

4. The method of any one of the preceding claims, wherein the advising is further based on a real-time clock.

5. The method of any one of the preceding claims, wherein the recording of the real-time data includes the recording of the time and/or type of meal eaten by the person

6. The method of any one of the preceding claims, wherein the advising is further based on the time and/or dosage of insulin taken by the person.

7. The method of any one of the preceding claims, further comprising providing further advice to the patient after receiving additional real-time data.

8. A portable electronic device for advising a person with diabetes, the portable electronic device comprising:
- a means for inputting data;
- a memory;
- a controller; and
- a display,
wherein:
- the inputting means is operable to receive one or more inputs from the person, and the inputting means is coupled to the controller such that the controller is operable to receive the one or more inputs from the person,
- the display is operable to display information to the person, and the controller is coupled to the display such that the controller is operable to send the information to the display,
- the memory is coupled to the controller and is operable to receive data from the controller, store data, and send data to the controller, wherein the memory stores a library of tasks, wherein each task is related to the therapy recommended for the person and the one or more tasks are a subset from a large group of potential tasks, the subset being specific to the therapy recommended to the person;
- the controller executes a computer program, wherein:
- the computer program receives personal data representing characteristics of the person related to the person's diabetic condition from the controller,
- the computer program receives real-time data related to the person's diabetic condition from the controller, and
- the computer program advises the person via the display to perform a task from the library based on the personal data and the real-time data,
**characterized in that** the tasks correspond to the characteristics of the portable electronic device **in that** tasks are omitted from the library if they are not appropriate for the portable electronic device.

9. The portable electronic device of claim 8, wherein the task being advised is injecting basal insulin, taking a blood glucose measurement, recording the person's meal data and time, taking medication, or visiting the doctor.

10. The portable electronic device of claim 8 or 9, wherein the portable electronic device is a cellular phone, a personal digital assistant, or a smart phone.

11. The portable electronic device of any one of claims 8 to 10, further comprising on a real-time clock in communication with the controller, wherein the computer program advises the person to perform a task from the library further based on the real-time clock.

12. The portable electronic device of any one of claims 8 to 11, wherein the recording of the real-time data includes recording of the time and/or type of meal eaten by the person

13. The portable electronic device of any one of claims 8 to 12, wherein the advising is further based on the time and/or dosage of insulin taken by the person.

## Patentansprüche

1. Verfahren zum Beraten einer Person mit Diabetes, wobei das Verfahren auf einem tragbaren elektronischen Gerät durchgeführt wird und das Verfahren umfasst:
- Einrichten, in einem Speicher des tragbaren elektronischen Geräts, einer Bibliothek von einer oder mehreren Aufgaben, wobei jede Aufgabe in der Bibliothek im Zusammenhang mit einer Therapie steht, die der Person mit Diabetes empfohlen wird, und die eine oder die mehreren Aufgaben eine Teilmenge aus einer großen Gruppe von möglichen Aufgaben sind, wobei die Teilmenge spezifisch für die Therapie ist, die der Person empfohlen wird;
- Aufzeichnen von persönlichen Daten, die über eine Eingabeeinrichtung des tragbaren elektronischen Geräts empfangen werden, wobei die persönlichen Daten Merkmale der Person darstellen, die im Zusammenhang mit dem diabetischen Zustand der Person stehen;
- Aufzeichnen von Echtzeitdaten, die über die Eingabeeinrichtung des tragbaren elektronischen Geräts empfangen werden, wobei die Echtzeitdaten im Zusammenhang mit dem diabetischen Zustand der Person stehen; und
- Beraten der Person, über einen Bildschirm des tragbaren elektronischen Geräts, eine Aufgabe aus der Bibliothek durchzuführen, basierend auf den persönlichen Daten und den Echtzeitdaten,
**dadurch gekennzeichnet, dass** die Aufgaben den Eigenschaften des tragbaren elektronischen Geräts dadurch entsprechen, dass Aufgaben aus der Bibliothek weggelassen werden, wenn sie für das tragbare elektronische Gerät nicht geeignet sind.

2. Verfahren nach Anspruch 1, wobei die angeratene Aufgabe ein Injizieren von Basalinsulin, Durchführen einer Blutzuckermessung, Aufzeichnen von Mahlzeitendaten und -zeit der Person, Einnehmen von Medikamenten oder Aufsuchen des Arztes ist.

3. Verfahren nach Anspruch 1 oder 2, wobei das tragbare elektronische Gerät ein Mobiltelefon, ein persönlicher digitaler Assistent oder ein Smartphone ist.

4. Verfahren nach einem der vorhergehenden Ansprüche, wobei das Beraten des Weiteren auf einer Echtzeituhr basiert.

5. Verfahren nach einem der vorhergehenden Ansprüche, wobei das Aufzeichnen der Echtzeitdaten das Aufzeichnen der Zeit und/oder der Art der von der Person verzehrten Mahlzeit enthält.

6. Verfahren nach einem der vorhergehenden Ansprüche, wobei das Beraten des Weiteren auf der Zeit und/oder der Dosis des von der Person eingenommenen Insulins basiert.

7. Verfahren nach einem der vorhergehenden Ansprüche, das des Weiteren das Erteilen weiterer Ratschläge an den Patienten nach Erhalten zusätzlicher Echtzeitdaten umfasst.

8. Tragbares elektronisches Gerät zum Beraten einer Person mit Diabetes, wobei das tragbare elektronische Gerät umfasst:
- eine Dateneingabeeinrichtung;
- einen Speicher;
- eine Steuervorrichtung; und
- einen Bildschirm,
wobei:
- die Eingabeeinrichtung betriebsfähig ist, eine oder mehrere Eingaben von der Person zu empfangen, und die Eingabeeinrichtung mit der Steuervorrichtung derart gekoppelt ist, dass die Steuervorrichtung betriebsfähig ist, die eine oder die mehreren Eingaben von der Person zu empfangen,
- der Bildschirm betriebsfähig ist, der Person Informationen anzuzeigen, und die Steuervorrichtung mit dem Bildschirm derart gekoppelt ist, dass die Steuervorrichtung betriebsfähig ist, die Informationen an den Bildschirm zu senden,
- der Speicher mit der Steuervorrichtung gekoppelt ist und betriebsfähig ist, Daten von der Steuervorrichtung zu empfangen, Daten zu speichern und Daten an die Steuervorrichtung zu senden, wobei der Speicher eine Bibliothek von Aufgaben speichert, wobei jede Aufgabe im Zusammenhang mit der Therapie steht, die der Person empfohlen wird, und die eine oder die mehreren Aufgaben eine Teilmenge aus einer großen Gruppe von möglichen Aufgaben sind, wobei die Teilmenge spezifisch für die Therapie ist, die der Person empfohlen wird;
- die Steuervorrichtung ein Computerprogramm ausführt, wobei:
- das Computerprogramm von der Steuervorrichtung persönliche Daten empfängt, die Merkmale der Person darstellen, die im Zusammenhang mit dem diabetischen Zustand der Person stehen,
- das Computerprogramm von der Steuervorrichtung Echtzeitdaten empfängt, die im Zusammenhang mit dem diabetischen Zustand der Person stehen, und
- das Computerprogramm der Person über den Bildschirm rät, eine Aufgabe aus der Bibliothek basierend auf den persönlichen Daten und den Echtzeitdaten durchzuführen,
**dadurch gekennzeichnet, dass** die Aufgaben den Eigenschaften des tragbaren elektronischen Geräts dadurch entsprechen, dass Aufgaben aus der Bibliothek weggelassen werden, wenn sie für das tragbare elektronische Gerät nicht geeignet sind.

9. Tragbares elektronisches Gerät nach Anspruch 8, wobei die angeratene Aufgabe ein Injizieren von Basalinsulin, Durchführen einer Blutzuckermessung, Aufzeichnen von Mahlzeitendaten und -zeit der Person, Einnehmen von Medikamenten oder Aufsuchen des Arztes ist.

10. Tragbares elektronisches Gerät nach Anspruch 8 oder 9, wobei das tragbare elektronische Gerät ein Mobiltelefon, ein persönlicher digitaler Assistent oder ein Smartphone ist.

11. Tragbares elektronisches Gerät nach einem der Ansprüche 8 bis 10, das des Weiteren eine Echtzeituhr umfasst, die mit der Steuervorrichtung kommuniziert, wobei das Computerprogramm der Person rät, eine Aufgabe aus der Bibliothek durchzuführen, die des Weiteren auf der Echtzeituhr basiert.

12. Tragbares elektronisches Gerät nach einem der Ansprüche 8 bis 11, wobei das Aufzeichnen der Echtzeitdaten das Aufzeichnen der Zeit und/oder der Art der von der Person verzehrten Mahlzeit enthält.

13. Tragbares elektronisches Gerät nach einem der Ansprüche 8 bis 12, wobei das Beraten des Weiteren auf der Zeit und/oder der Dosis des von der Person eingenommenen Insulins basiert.

## Revendications

1. Procédé destiné à apporter des conseils à une personne atteinte de diabète, le procédé étant effectué sur un appareil électronique portable et le procédé comprenant :
- l'établissement, dans une mémoire du dispositif électronique portable, d'une bibliothèque d'une ou plusieurs tâches, chaque tâche de la bibliothèque étant liée à un traitement recommandé à la personne atteinte de diabète et la ou les tâches étant un sous-ensemble d'un groupe considérable de tâches potentielles, le sous-ensemble étant spécifique au traitement recommandée à la personne ;
- l'enregistrement de données personnelles reçues par l'intermédiaire d'un organe de saisie du dispositif électronique portable, les données personnelles représentant des caractéristiques de la personne liées à son état diabétique ;
- l'enregistrement de données en temps réel reçues par l'intermédiaire de l'organe de saisie du dispositif électronique portable, les données en temps réel étant liées au état diabétique de la personne ; et
- l'apport de conseils à la personne, par l'intermédiaire d'un écran de l'appareil électronique portable, à effectuer une tâche issue de la bibliothèque, sur la base des données personnelles et des données en temps réel,
**caractérisé en ce que** les tâches correspondent aux caractéristiques du dispositif électronique portable **en ce que** les tâches sont omises de la bibliothèque si elles ne conviennent pas au dispositif électronique portable.

2. Procédé selon la revendication 1, la tâche conseillée consistant à injecter de l'insuline basale, à relever une mesure de la glycémie, à enregistrer les données et l'heure des repas de la personne, à prendre des médicaments ou à consulter un médecin.

3. Procédé selon la revendication 1 ou 2, le dispositif électronique portable étant un téléphone cellulaire, un assistant numérique personnel ou un téléphone intelligent.

4. Procédé selon l'une quelconque des revendications précédentes, l'apport de conseils étant en outre basé sur une horloge en temps réel.

5. Procédé selon l'une quelconque des revendications précédentes, l'enregistrement des données en temps réel comportant l'enregistrement de l'heure et/ou du type de repas consommé par la personne.

6. Procédé selon l'une quelconque des revendications précédentes, l'apport de conseils étant en outre basé sur l'heure et/ou le dosage d'insuline pris par la personne.

7. Procédé selon l'une quelconque des revendications précédentes, comprenant en outre la fourniture de conseils supplémentaires au patient après réception de données en temps réel supplémentaires.

8. Dispositif électronique portable destiné à apporter des conseils à une personne atteinte de diabète, le dispositif électronique portable comprenant :
- un organe de saisie de données ;
- une mémoire ;
- un dispositif de commande ; et
- un écran ,
dans lequel :
- l'organe de saisie est capable de recevoir une ou plusieurs saisies émanant de la personne, et l'organe de saisie est couplé au dispositif de commande de telle sorte que le dispositif de commande est capable de recevoir la ou les saisies émanant de la personne,
- l'écran est capable d'afficher des informations à la personne, et le dispositif de commande est couplé à l'écran de telle sorte que le dispositif de commande est capable d'envoyer les informations à l'écran,
- la mémoire est couplée au dispositif de commande et est capable de recevoir des données émanant du dispositif de commande, stocker des données et envoyer des données au dispositif de commande, la mémoire stockant une bibliothèque de tâches, chaque tâche étant liée au traitement recommandé pour la personne et la ou les tâches représentant un sous-ensemble d'un groupe considérable de tâches potentielles, le sous-ensemble étant spécifique au traitement recommandé à la personne ;
- le dispositif de commande exécute un programme informatique, dans lequel :
- le programme informatique reçoit des données personnelles représentant les caractéristiques de la personne liées à son état diabétique en provenance du dispositif de commande,
- le programme informatique reçoit des données en temps réel liées au état diabétique de la personne en provenance du dispositif de commande, et
- le programme informatique apporte des conseils à la personne, par l'intermédiaire de l'écran, à effectuer une tâche issue de la bibliothèque sur la base des données personnelles et des données en temps réel,
**caractérisé en ce que** les tâches correspondent aux caractéristiques du dispositif électronique portable **en ce que** les tâches sont omises de la bibliothèque si elles ne conviennent pas au dispositif électronique portable.

9. Dispositif électronique portable selon la revendication 8, la tâche conseillée consistant à injecter de l'insuline basale, à relever une mesure de la glycémie, à enregistrer les données et l'heure des repas de la personne, à prendre des médicaments ou à consulter un médecin.

10. Dispositif électronique portable selon la revendication 8 ou 9, le dispositif électronique portable étant un téléphone cellulaire, un assistant numérique personnel ou un téléphone intelligent.

11. Dispositif électronique portable selon l'une quelconque des revendications 8 à 10, comprenant en outre une horloge en temps réel en communication avec le dispositif de commande, le programme informatique conseillant à la personne d'effectuer une tâche issue de la bibliothèque sur la base de l'horloge en temps réel.

12. Dispositif électronique portable selon l'une quelconque des revendications 8 à 11, l'enregistrement des données en temps réel comportant l'enregistrement de l'heure et/ou du type de repas consommé par la personne.

13. Dispositif électronique portable selon l'une quelconque des revendications 8 à 12, l'apport de conseils étant en outre basé sur l'heure et/ou le dosage d'insuline pris par la personne.
